# EUROPÄISCHE PATENTSCHRIFT

(11) **EP 1 975 610 B1**
(45) Veröffentlichungstag und Bekanntmachung des Hinweises auf die Patenterteilung: **07.01.2009**
(21) Anmeldenummer: 07104960.5
(22) Anmeldetag: 27.03.2007
(51) Int. Cl.: G01N 33/487, B65D 83/08, G01N 33/49, G01N 35/00, B65D 83/02, G01N 21/77

(54) **Analysegerät mit austauschbarem Testelementmagazin**
Analysis device with exchangeable test element magazine
Instrument d'analyse avec magasin échangeable d'éléments de test

(43) Veröffentlichungstag der Anmeldung: 01.10.2008
(73) Patentinhaber: F. Hoffmann-La Roche AG, 4070 Basel (CH); Roche Diagnostics GmbH, 68305 Mannheim (DE)
(72) Erfinder: List, Hans, 64754 Hesseneck-Kailbach (DE); Zimmer, Volker, 67229 Laumersheim (DE); Frey, Stephan Michael, 64347 Griesheim (DE)
(74) Vertreter: Hörschler, Wolfram Johannes

(56) Entgegenhaltungen:
- WO-A-03/082091
- WO-A-20/06047135
- WO-A-20/06076721
- US-A1- 2003 089 730
- US-B2- 6 908 008

## Beschreibung

### Gebiet der Erfindung

Die Erfindung betrifft ein Messsystem mit einem Analysegerät und einem mit dem Analysegerät verbindbaren Magazin zur Aufnahme mindestens eines Verbrauchselements. Derartige Messsysteme und Analysegeräte werden beispielsweise eingesetzt, um flüssige Proben auf mindestens einen darin enthaltenen Analyten zu analysieren. Alternativ kommen derartige Messsysteme beispielsweise auch als Lanzettensysteme mit einer Vielzahl auswechselbarer Einweglanzetten zum Einsatz. Messsysteme dieser Art werden insbesondere im Bereich der Medizintechnik, beispielsweise bei der Blutglucose-Überwachung, oder im Bereich der chemischen Analytik, der biologischen Analytik oder der Umweltanalytik eingesetzt.

### Stand der Technik

Die Überwachung der Blutglucose-Konzentration ist für Diabetiker ein wesentlicher Bestandteil des Tagesablaufs. Dabei muss die Blutglucose-Konzentration in der Regel schnell und zuverlässig mehrmals am Tag bestimmt werden, um gegebenenfalls entsprechende medizinische Maßnahmen ergreifen zu können. Um den Tagesablauf des Diabetikers nicht mehr als nötig einzuschränken, werden dabei häufig entsprechende mobile Geräte eingesetzt, welche einfach zu transportieren und zu handhaben sein sollten, so dass die Messung der Blutglucose-Konzentration beispielsweise am Arbeitsplatz oder in der Freizeit erfolgen kann.

Derzeit sind verschiedene mobile Geräte am Markt, welche teilweise nach unterschiedlichen Messmethoden funktionieren. Dabei kommen verschiedene Diagnoseverfahren zum Einsatz, beispielsweise optische oder auch elektrochemische Messverfahren. Hierfür können beispielsweise Testelemente, zum Beispiel Teststreifen, zum Einsatz kommen, auf welche eine entsprechende Probe aufgebracht wird. Ein Beispiel derartiger Teststreifen (in diesem Fall elektrochemischer Teststreifen) ist in US 5,286,362 dargestellt. Beispiele optischer Messsysteme sind in WO 01/48461 beschrieben.

Verbrauchselemente, beispielsweise Testelemente (insbesondere Teststreifen), bilden somit ein wesentliches Element derartiger Messsysteme, insbesondere der tragbaren Messsysteme. Typischerweise werden von einem Diabetiker ca. 5 bis 7 Testelemente pro Tag benötigt. Essentiell ist dabei, dass die Teststreifen sauber und trocken aufbewahrt werden, um nicht durch eine Verschmutzung bzw. Einwirkung von Feuchtigkeit die Messung der Blutglucose-Konzentration zu verfälschen. Ähnliches gilt beispielsweise auch für Einweglanzetten als Verbrauchselemente, welche ebenfalls insbesondere steril gelagert werden müssen.

Zu diesem Zweck werden die Verbrauchselemente üblicherweise in entsprechenden Behältnissen aufbewahrt, um anschließend vom Benutzer für eine Messung dem Behältnis entnommen und in ein entsprechendes Analysegerät eingegeben zu werden. Derartige Systeme sind beispielsweise aus US 2002/0170823 A1 bekannt. Zum Aufbewahren und Ausgeben der Teststreifen werden teilweise auch Magazinsysteme eingesetzt. Beispiele derartiger Systeme sind aus US 2003/O116583 A1, US 6,908,008 B2, EP-0 640 393 B1 und US 4,911,344 bekannt. In diesen Systemen werden mehrere Teststreifen in einem Magazin gelagert. EP 1 488 736 A1 beschreibt darüber hinaus ein System, in welchem anstelle einzelner Teststreifen eine Bandkassette eines einzelnen langen Teststreifens mit mehreren Testfeldern aufgenommen ist.

Neben Systemen, bei denen Magazin und Messgerät als getrennte Einheiten eingesetzt werden, existieren auch integrierte Systeme, bei welchen mehrere Teststreifen nicht nur in einem Magazin gelagert werden, sondern welche gleichzeitig auch die Möglichkeit einer Auswertung dieser Teststreifen bieten. Beispiele derartiger integrierter Systeme sind in US 6,827,899 B2 oder in US 6,159,424 offenbart.

Dabei sind verschiedene Formen der Bevorratung der Testelemente bekannt, bei denen eine Abschottung gegen äußere Einflüsse entweder durch das Magazin selbst oder durch das Analysegerät erfolgt. Im ersten Fall muss das Magazin selbst alle Mittel zur Abdichtung und die Möglichkeit zur Öffnung der Abdichtung bereithalten, was vergleichsweise komplexe und damit teure Magazine zur Folge hat. Ein Beispiel eines derartigen Systems ist das in EP 1 488 736 A1 beschriebene Bandkassetten-System oder auch Systeme mit aufgesiegelten Folien.

Im zweiten beschriebenen Fall, in welchem die Abdichtung durch das Analysegerät erfolgt, muss das Analysegerät eine dichte Kammer aufweisen, die sich vom Anwender beim Magazinwechsel öffnen und wieder dicht verschließen lassen muss. Ein Beispiel eines derartigen Systems zeigt US 6,827,899 B2. Nachteilig an derartigen Systemen ist jedoch, dass diese entweder technisch sehr aufwändig sind, oder dass diese eine vergleichsweise große und gut für den Anwender zugängliche Dichtung aufweisen, welche somit durch Fehlanwendungen oder mangelnde Sorgfalt oder Sauberkeit in ihrer Funktion beeinträchtigt werden kann. Zudem ermöglichen viele der genannten Dichtprinzipien, wie beispielsweise auch das in US 6,827,899 B2 beschriebene Prinzip, keinen Austausch eines Magazins, so dass nach Verbrauch der Testelemente das System vollständig ausgetauscht werden muss.

Aus der bereits erwähnten US 6,908,008 B2 ist eine Testvorrichtung bekannt, bei welcher Teststreifen in einem Magazin gelagert werden. Dabei ist das Magazin derart ausgestaltet, dass dies an seiner Oberseite (Ausgabeseite) offen ist und eine um diese Öffnung umlaufende Gummidichtung aufweist. Diese drückt im geschlossenen Zustand des Magazins gegen eine entsprechende Dichtfläche des Testgeräts. Zum Entnehmen eines Teststreifens wird das Magazin von der Dichtfläche abgehoben, so dass über einen Schieber ein Teststreifen aus dem Magazin ausgeschoben werden kann. die in US 6,908,008 B2 gezeigte Anordnung ist jedoch technisch vergleichsweise aufwändig, da das Abheben des Magazins von der Dichtfläche des Testgeräts eine entsprechende Mechanik erfordert. Zudem werden vergleichsweise hohe Anforderungen an die Dichtigkeit des gesamten Testsystems gestellt, da während des Abhebens und der Ausgabe eines Teststreifens eine vergleichsweise große Öffnung entsteht, durch welche Feuchtigkeit in das Innere des Magazins eindringen könnte.

Aus WO 2006/047135 ist ein Teststreifenspender mit auswechselbarem Teststreifenmagazin bekannt, der bei Betätigung eines Handschiebers ein Siegel von der Ausgabeöffnung weg schiebt, einen Teststreifen aus dem Magazin ausgibt und dann die Ausgabeöffnung wieder mit dem Siegel verschließt.

### Aufgabe der Erfindung

Aufgabe der Erfindung ist daher, ein Messsystem bereitzustellen, welches die oben beschriebenen Nachteile des Standes der Technik zumindest weitgehend vermeidet. Insbesondere soll das Messsystem neben einem Analysegerät mindestens ein Magazin umfassen, welches austauschbar ist und welches auf einfache und schnelle Weise dicht mit dem Analysegerät verbunden werden kann.

### Beschreibung der Erfindung

Diese Aufgabe wird durch ein Messsystem mit den Merkmalen des unabhängigen Anspruchs 1 gelöst. Vorteilhafte Weiterbildungen, welche einzeln oder in Kombination verwirklicht sein können, sind in den abhängigen Ansprüchen dargestellt.

Es wird ein Messsystem vorgeschlagen, welches beispielsweise von seiner Funktion her den oben beschriebenen Geräten entsprechen kann. Beispielsweise kann es sich dabei um ein Messsystem zur Analyse von Blut oder anderen Körperflüssigkeiten auf einen oder mehrere Analyten, wie beispielsweise Blutglucose, handeln. Alternativ oder zusätzlich kann es sich bei diesem "Messsystem" auch um ein Probennahmesystem handeln, welches mindestens eine Probennahmefunktion aufweist, beispielsweise die Bereitstellung eines Blutstropfens durch Perforation einer Hautpartie eines Patienten. Insoweit ist der Begriff "Messsystem" weit zu fassen.

Das Messsystem weist ein Analysegerät mit mindestens einer Messfunktion und/oder Probenentnahmefunktion auf. Diese Messfunktion kann beispielsweise die Analyse einer flüssigen Probe mittels eines Testelements umfassen. Die Probennahmefunktion kann beispielsweise die beschriebene Perforation einer Hautpartie mittels eines Stechelements, beispielsweise einer Lanzette umfassen. Insofern ist der Begriff "Analysegerät" weit zu fassen.

Weiterhin umfasst das Messsystem mindestens ein Magazin zur Aufnahme mindestens eines Verbrauchselements. Die Verbrauchselemente können der Funktion des Analysegeräts angepasst sein und können somit beispielsweise Testelemente, insbesondere Teststreifen (beispielsweise optische und/oder elektrochemische Teststreifen, beispielsweise gemäß dem eingangs beschriebenen Stand der Technik) umfassen. Alternativ oder zusätzlich können die Verbrauchselemente Stechhilfen umfassen, beispielsweise Lanzetten, insbesondere Einweglanzetten (beispielsweise Einweglanzetten, wie sie in EP 1 203 563 A2 und/oder in EP 1 333 756 B1 beschrieben sind).

Das Magazin weist ein Magazingehäuse auf, welches zumindest im Wesentlichen eine Abschottung der Verbrauchselemente gegenüber Umwelteinflüssen, wie insbesondere Verschmutzung und/oder Feuchtigkeit, gewährleisten sollte. Das Magazingehäuse weist mindestens eine Öffnung auf, wobei die mindestens eine Öffnung die Entnahme und/oder Ausgabe eines Verbrauchselements ermöglicht. Das Messsystem weist einen Verschluss mit mindestens einem Dichtelement (im Folgenden auch "geräteseitiges Dichtelement genannt) auf, um die Öffnung zu verschließen.

Das Magazin ist als auswechselbares Magazin ausgestaltet, welches mit dem Analysegerät verbindbar ist. Zu diesem Zweck können das Analysegerät und/oder das Magazin insbesondere entsprechende Verbindungselemente umfassen, beispielsweise Bajonettverschlüsse, Haken, Riegel oder andere Arten von Sperrelementen. Bevorzugt ist es, wenn das Analysegerät eine Magazinaufnahme aufweist, insbesondere einen Magazinschacht oder ein Magazinfach, welche bzw. welches eingerichtet ist, um das Magazin aufzunehmen und dort entsprechend mit dem Analysegerät zu verbinden.

Messsysteme dieser Art entsprechen beispielsweise dem in US 6,908,008 B2 beschriebenen Messsystem. Im Unterschied zu der aus dieser Druckschrift bekannten Konstruktion ist das Dichtelement jedoch erfindungsgemäß derart eingerichtet, dass dieses eine Doppelfunktion erfüllt: Zum einen ist das Dichtelement (bzw. der gesamte Verschluss) eingerichtet, um mindestens ein Verbrauchselement aus der Öffnung auszugeben. Beispielsweise kann das Dichtelement zu diesem Zweck in eine erste Öffnung des Magazins eingeschoben werden, um dann ein Verbrauchselement aus einer zweiten Öffnung auszustoßen. In diesem Fall kann das Dichtelement beispielsweise einen Schieber oder Stößel umfassen. Auch andere Arten der Ausgabe sind jedoch denkbar, beispielsweise eine Greiferkonstruktion, mittels derer das Verbrauchselement aus der Öffnung herausgezogen wird.

Zum anderen ist das Dichtelement (bzw. der gesamte Verschluss) jedoch auch so eingerichtet, dass dieses in einem geschlossenen Zustand des Magazins in der mindestens einen Öffnung aufgenommen ist und dabei derart mit dieser Öffnung bzw. dem Magazin im Bereich der Öffnung zusammenwirkt, dass diese Öffnung verschlossen wird. Unter "verschlossen" ist dabei eine Abschottung gegenüber Verschmutzungen und Luftfeuchtigkeit zu verstehen, welche jedoch nicht notwendigerweise vollständig luftdicht wirkt. Die Rate, mit der Feuchtigkeit durch die verschlossene Öffnung eindringt, sollte (unter Berücksichtigung ggf. im Magazin aufgenommener Trockenmittel, derart eingestellt sein, dass die Verbrauchselemente über eine übliche Lagerdauer (d.h. vor Ablauf eines Verfallsdatums oder üblicherweise wenige Tage bis Wochen) nicht wesentlich in ihrer Funktion beeinträchtigt werden. Bei Lanzettensystemen sind dabei andere Anforderungen an die Dichtigkeit zu stellen, da hier die Sterilität eine größere Rolle spielt als die Trockenheit.

Im Gegensatz zum bekannten Stand der Technik bietet das beschriebene Messsystem somit den Vorteil, dass einerseits das Magazin mit den Verbrauchselementen ausgetauscht werden kann, so dass bei leerem Magazin lediglich ein Austausch des Magazins erfolgen kann, wobei das eigentliche Analysegerät weiterverwendet werden kann. Dabei ist der eigentliche Verschluss im Analysegerät vorgesehen, so dass komplexe Bauteile dieses Verschlusses nicht mit dem Magazin entsorgt werden brauchen, sondern als wiederverwendbare Bauteile im Analysegerät verbleiben können. Hierdurch lassen sich erhebliche Kosten einsparen. Die Betriebskosten dieser Systeme, welche insbesondere als tragbare, leichte und kostengünstige Messsysteme ausgestaltet sein können, lassen sich auf ein Minimum reduzieren. Gleichzeitig lassen sich Fehlfunktionen und Fehlbedienungen dadurch vermeiden, dass der Verschluss beispielsweise innen liegend in einem in einem Gehäuse des Analysegerätes (beispielsweise im Inneren eines Magazinschachtes oder einer Magazinaufnahme) angeordnet sein kann, vorzugsweise unzugänglich für einen Benutzer.

Das Dichtelement und die mindestens eine Öffnung wirken also erfindungsgemäß im geschlossenen Zustand des Messsystems zusammen, um die mindestens eine Öffnung abzudichten. Dieses Zusammenwirken kann auf verschiedene Weisen erfolgen. Beispielsweise können die Öffnung bzw. das Magazin im Bereich der Öffnung und/oder das Dichtelement jeweils Dichtflächen aufweisen, die im geschlossenen Zustand gegeneinander gedrückt werden, um auf diese Weise eine dichtende Wirkung zu erzielen. Alternativ oder zusätzlich können auch das Dichtelement und/oder die Öffnung bzw. das Magazin im Bereich der Öffnung zusätzliche Dichtelemente aufweisen, beispielsweise elastische Dichtelemente. Zu diesem Zweck können das Dichtelement und/oder die Öffnung beispielsweise ganz oder teilweise verformbar, insbesondere elastisch, ausgestaltet sein, um bei entsprechendem Aufeinanderpressen die dichtende Wirkung zu erzielen. Auch eine Kombination der Techniken ist möglich, beispielsweise die Kombination einer glatten Dichtfläche mit einem verformbaren Dichtelement, wobei die Verteilung dieser Elemente auf das Dichtelement bzw. die Öffnung beliebig wählbar ist.

Besonders bevorzugt ist es jedoch, wenn das Dichtelement eine Dichtfläche, vorzugsweise eine glatte Dichtfläche, aufweist. Als Gegenstück kann die Öffnung dann ein verformbares magazinseitiges Dichtelement aufweisen, welches mit der Dichtfläche zusammenwirkt. Beispielsweise kann die Öffnung mindestens einen Dichtspalt, insbesondere eine Dichtlippe aufweisen. Unter einer "Dichtlippe" ist dabei beispielsweise eine um die Öffnung umlaufende Gummidichtung zu verstehen, in welche das Dichtelement eingeschoben werden kann.

Weitere bevorzugte Ausführungsformen betreffen das Dichtelement selbst. So hat es sich beispielsweise, um die dichtende Wirkung zu verbessern, als vorteilhaft erwiesen, wenn das Dichtelement in einer Einschubsrichtung in die Öffnung (das heißt in der Richtung, in welcher das Dichtelement durch den Verschluss in die Öffnung eingeschoben wird) zumindest teilweise (das heißt beispielsweise in einem Abschnitt des Dichtelements) einen sich verjüngenden Querschnitt aufweist. Auf diese Weise kann durch die durch den Verschluss ausgeübte Kraft eine Kraftkomponente ausgebildet werden, über die das Dichtelement gegen die Wandung der Öffnung gepresst wird.

Dabei kann das Dichtelement derart ausgestaltet sein, dass dies beispielsweise in einigen Bereichen mit konstantem Querschnitt ausgebildet ist, in anderen Bereichen hingegen mit dem beschriebenen, sich verjüngenden Querschnitt (zum Beispiel einem konischen Querschnitt). Dadurch kann beispielsweise der erste, mit konstantem Querschnitt versehene Abschnitt zum Ausgeben oder Ausschieben eines Verbrauchselements genutzt werden, um anschließend, mit ein- und derselben Bewegung, den konischen Abschnitt in die Öffnung einzuführen und ein Verschließen der Öffnung zu bewerkstelligen.

Der Verschluss kann insbesondere derart eingerichtet sein, dass mittels des Dichtelements das bzw. ein Verbrauchselement aus dem Magazin in eine Anwendungsposition befördert wird. Beispielsweise kann in dieser Anwendungsposition mittels des als Teststreifen ausgebildeten Gebrauchselements die Konzentration eines Analyten in einer flüssigen Probe bestimmt werden. Alternativ oder zusätzlich kann mittels eines als Lanzette ausgebildeten Verbrauchselements in der Anwendungsposition eine Perforation einer Hautpartie vorgenommen werden, um einen Blutstropfen zu generieren. Auch andere Arten von Anwendungen sind jedoch denkbar.

Wenn das Verbrauchselement mittels des Dichtelements aus dem Magazin in eine Anwendungsposition befördert wird, so können auch andere Aufgaben durch das Dichtelement wahrgenommen werden. Beispielsweise kann das Dichtelement auch dazu dienen, das Verbrauchselement nach Verwendung aus der Anwendungsposition in eine Entsorgungsposition und/oder in eine Entsorgungsvorrichtung zu befördern. Beispielsweise kann zu diesem Zweck das Messsystem ein Abfallbehälter aufweisen, welcher derartige verbrauchte Verbrauchselement aufnehmen kann. Dieser Abfallbehälter kann beispielsweise derart eingerichtet sein, dass dieser abnehmbar mit dem Messsystem und/oder Analysegerät verbunden ist, um eine regelmäßige Entleerung zu gewährleisten. Alternativ oder zusätzlich kann der Abfallbehälter auch Bestandteil des Magazins sein. Anstelle eines Abfallbehälters können verbrauchte Verbrauchselemente auch unmittelbar ausgeworfen werden. Verschiedene Ausgestaltungen sind denkbar.

Neben den oben beschriebenen Funktionen der Ausgabe eines Verbrauchselements und der Abdichtung der Öffnung kann das Dichtelement auch weitere Aufgaben bzw. Funktionen übernehmen. Diese Funktionen können beispielsweise mit der Ausgabe des Verbrauchselements zusammenhängen, so dass beispielsweise Greifer, Schieber oder ähnliches von dem Dichtelement umfasst sein können (siehe auch oben). Alternativ oder zusätzlich kann das Dichtelement auch weitere elektrische, optische oder mechanische Funktionen übernehmen, welche auf die Art und Funktionsweise des Verbrauchselements abgestimmt sein können.

So kann insbesondere das Dichtelement einen Mantel mit mindestens einer Dichtfläche zum Abdichten der Öffnung umfassen, sowie weiterhin, im Inneren des Dichtelements, einen axial im Dichtelement verlaufenden Applikationskanal. In diesem Applikationskanal, welcher Applikationskanal mit beliebigem Querschnitt ausgebildet sein kann, können verschiedene funktionelle Elemente aufgenommen sein.

So kann beispielsweise in dem Applikationskanal mindestens ein elektrischer Kontakt (beispielsweise eine Zuleitung) zur Kontaktierung eines Verbrauchselements aufgenommen sein. Auf diese Weise lassen sich beispielsweise über das Dichtelement elektrochemische Teststreifen kontaktieren, um eine entsprechende Messung durchzuführen.

Alternativ oder zusätzlich kann in dem Applikationskanal auch eine optische Vorrichtung aufgenommen sein. Diese optische Vorrichtung kann beispielsweise eine Lichtquelle und/oder einen Lichtwellenleiter umfassen, beispielsweise um einen optischen Teststreifen anzuregen und/oder von dem optischen Teststreifen imitiertes oder auf andere Weise ausgesandtes Licht zu sammeln und einer Detektionsvorrichtung zuzuführen. Auch andere Arten optischer Vorrichtungen sind denkbar.

Als dritte, alternativ oder zusätzlich implementierbare Möglichkeit kann in dem mindestens einen Applikationskanal auch eine mechanische Vorrichtung implementiert sein. Beispielsweise kann diese mechanische Vorrichtung eine Vorrichtung zum Handhaben der Verbrauchselemente umfassen. Als ein Beispiel kann hier eine Vorrichtung genannt sein, welche eine mechanische Bewegung durchführt, um Testelemente elektrisch zu kontaktieren. Dies kann beispielsweise von Vorteil sein, wenn Testelemente mit sehr dünnen Kontakten verwendet werden, welche auf diese Weise beispielsweise schonend kontaktiert werden können. Eine andere, alternativ oder zusätzlich einbringbare mechanische Funktion besteht darin, einen Antriebsstößel oder eine andere Antriebsvorrichtung in dem Applikationskanal anzuordnet, mittels derer ein Lanzettensystem betreibbar ist. Beispielsweise können in dem mindestens einen Magazin Einweglanzetten aufgenommen sein, welche durch das Dichtelement in eine Applikationsposition gebracht werden, in welcher diese dann durch den Antriebsstößel, welcher beispielsweise beweglich zum restlichen Dichtelement innerhalb des Applikationskanals gelagert ist, zu einer schnellen Perforationsbewegung angetrieben werden. Auch andere Arten der Ausgestaltung des Dichtelements sind denkbar.

### Ausführungsbeispiele

Weitere Einzelheiten und Merkmale der Erfindung ergeben sich aus der nachfolgenden Beschreibung von bevorzugten Ausführungsbeispielen in Verbindung mit den Unteransprüchen. Die Erfindung ist jedoch nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigt:
- Figur 1: einen Ausschnitt eines ersten Ausführungsbeispiels eines erfindungsgemäßen Messsystems mit einem Magazin im geöffneten Zustand;
- Figur 2: das Messsystem gemäß Figur 1 im geschlossenen Zustand;
- Figur 3: ein zu Figur 1 alternatives Ausführungsbeispiel mit einem konischen Dichtelement;
- Figur 4: das Messsystem gemäß Figur 3 in geschlossenem Zustand;
- Figur 5: ein weiteres Ausführungsbeispiel eines Messsystems mit einem optischen Detektor und einem Abfallbehälter;
- Figur 6: ein Ausführungsbeispiel eines Messsystems mit einem Lanzettensystem und einem hohlen Dichtelement;
- Figuren 7 und 8: Beispiele des Zusammenwirkens eines Lanzettenstößels mit einem Lanzettensystem;
- Figur 9: ein Ausführungsbeispiel eines Messsystems mit kombinierten Verbrauchselementen; und
- Figur 10: ein Ausführungsbeispiel eines Messsystems mit einem Trommelmagazin.

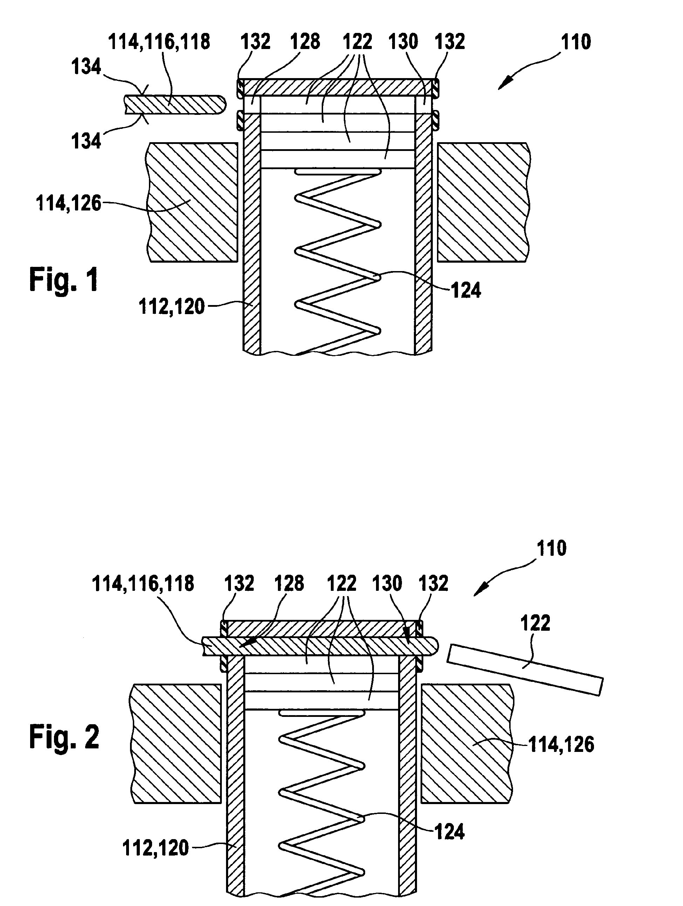

In Figur 1 ist in stark vereinfachter Darstellung ein Ausschnitt eines erfindungsgemäßen Messsystems 110 gemäß einem ersten Ausführungsbeispiel dargestellt. Das Messsystem 110 weist ein Magazin 112 und ein Analysegerät 114 auf. Dabei ist in dieser vereinfachten Darstellung von dem Analysegerät 114 lediglich ein Teil eines Verschlusses 116 mit einem Dichtelement 118 dargestellt. Für die übrigen Funktionen des Analysegeräts 114 und Beispiele eines Aufbaus kann beispielsweise auf die Beschreibung der weiteren Ausführungsbeispiele (siehe unten) verwiesen werden.

Das Magazin 112 weist ein Magazingehäuse 120 auf. Dieses Magazingehäuse ist im Wesentlichen als dichtes Gehäuse ausgestaltet und dient der Aufnahme mehrerer parallel aufeinander gestapelter Testelemente 122, welche in diesem Ausführungsbeispiel als Teststreifen ausgestaltet sind. Auch andere Arten von Verbrauchselementen sind jedoch möglich.

Das Magazin 112 ist dabei in dem dargestellten Ausführungsbeispiel als Stangen- oder Reihenmagazin ausgestaltet und verfügt über eine Magazinfeder 124, welche den Stapel der Testelemente 122 mit einer Kraft beaufschlagt.

Das Magazin 112 ist in diesem Beispiel ausgestaltet, um in einen (in Figur 1 lediglich symbolisch angedeuteten) Magazinschacht 126 des Analysegeräts 114 eingeschoben zu werden. Auch andere Ausgestaltungen der Verbindung des Magazins 112 mit dem Analysegerät 114 sind jedoch denkbar.

Das ansonsten im Wesentlichen dichte Magazingehäuse 120 weist an seinem oberen Ende zwei Öffnungen 128, 130 auf. Entsprechend der Ausgestaltung der Testelemente 122 als Teststreifen können diese Öffnungen 128, 130, insbesondere die Öffnung 130, welche als Ausgabeöffnung dient, als Öffnungsspalte ausgestaltet sein.

Weiterhin weist das Magazin 112 gemäß dem Ausführungsbeispiel in Figur 1 magazinseitige Dichtelemente 132 auf. Diese magazinseitigen Dichtelemente sind in dem dargestellten Ausführungsbeispiel als um die Öffnung 128 umlaufende Dichtlippen ausgebildet, welche jedoch im geöffneten Zustand nicht fest aufeinander liegen und die Öffnung 128 nur unwesentlich verengen. Alternativ zu einfachen Dichtlippen können auch andere Arten umlaufender Dichtungen verwendet werden, beispielsweise Dichtringe mit entsprechender Gestalt, Schaumstoffdichtungen oder ähnliche Dichtungen. Auch eine einfache glatte Öffnung, d.h. eine Ausgestaltung ohne zusätzliche magazinseitige Dichtelemente, ist denkbar.

Als Gegenstück zu diesen magazinseitigen Dichtelementen 132 weist das (geräteseitige) Dichtelement 118, welches hier als einfache Stange oder Dichtschieber ausgebildet ist, Dichtflächen 134 auf, welche als glatte Flächen ausgebildet sind. Beispielsweise kann das Dichtelement 118 eine Stange mit konstantem rechteckigem Querschnitt sein, wobei dieser rechteckige Querschnitt beispielsweise im Wesentlichen dem Querschnitt der Öffnungen 128, 130 entspricht, bzw. nur unwesentlich kleiner ist als diese. Alternativ kann der Querschnitt auch geringfügig größer gewählt werden, so dass das Dichtelement 118 mit den Öffnungen 128, 130 im geschlossenen Zustand eine Presspassung bildet.

Die beiden Figuren 1 und 2 zeigen unterschiedliche Stellungen des Messsystems 110. In Figur 1 ist eine geöffnete Stellung gezeigt, bei welcher das Dichtelement 118 aus den Öffnungen 128, 130 herausgezogen ist, wodurch diese Öffnungen 128, 130 freigegeben sind. In dieser Stellung kann beispielsweise das Magazin 112 ausgewechselt werden. Weiterhin kann in dieser Stellung das oberste Testelement 122 durch die Magazinfeder 124 nachgeschoben werden und gegen die Innenseite des Magazindeckels gedrückt werden.

In Figur 2 ist dargestellt, wie das geräteseitige Dichtelement 118 durch einen (in Figur 1 und 2 nicht dargestellten) Mechanismus des Verschlusses 116 zunächst in die linke Öffnung 128 eingeschoben wird, dabei das oberste Testelement 122 aus der rechten Öffnung 130 ausgibt und dann die rechte Öffnung 130 verschließt. Für dieses Verschließen wirken die Dichtflächen 134 (siehe Figur 1) mit den magazinseitigen Dichtelementen 132 zusammen. Nach dem Ausgeben verbleibt dann das Dichtelement 118 in der in Figur 2 dargestellten geschlossenen Position. Somit sind die Öffnungen 128, 130 lediglich kurzfristig während des Ausgebens eines Testelements 122 (d.h. typischerweise nicht länger als für einige Sekunden) geöffnet und werden anschließend für eine längere Zeit, in der das Messsystem 110 unbenutzt ist, durch das Dichtelement 118 verschlossen.

Die magazinseitigen Dichtelemente 132, welche, wie oben beschrieben, als Dichtlippen ausgestaltet sein können, können vorformbare bzw. flexible Eigenschaften aufweisen und beispielsweise aus Gummi oder einem anderen Kunststoff gefertigt sein. Dementsprechend schließt das geräteseitige Dichtelement 118 in dem in Figur 2 dargestellten geschlossenen Zustand das Magazin 112 vollständig und schirmt dadurch die im Inneren des Magazins 112 aufgenommenen Testelemente 122 gegenüber Verschmutzungen und Feuchtigkeitseinwirkungen ab. Das oberste dieser Testelemente 122 wird dabei durch die Magazinfeder 124 gegen die untere glatte Dichtfläche 134 des geräteseitigen Dichtelements 118 gedrückt.

In dem geschlossenen Zustand, welcher in Figur 2 dargestellt ist, trägt das geräteseitige Dichtelement 118 vorzugsweise auch zu einer Verriegelung des Magazins 112 im Magazinschacht 126 bei. Das Magazin 112 ist dadurch fest mit dem Analysegerät 114 verbunden. Um auch in dem in Figur 1 dargestellten (in der Regel nur kurzzeitig auftretenden) geöffneten Zustand das Magazin 112 im Magazinschacht 126 zu fixieren, können darüber hinaus zusätzliche Verriegelungselemente vorgesehen sein.

In Figur 2 ist symbolisch der Ausstoß eines der Testelemente 122 durch das geräteseitige Dichtelement 118 dargestellt. Diese Darstellung ist stark vereinfacht. Bei dem vorgeschlagenen Messsystem mit der mindestens einen Messfunktion kann dieses Testelement 122 beispielsweise auch an eine Messfunktion übergeben werden, um beispielsweise elektrisch kontaktiert zu werden oder anderen Zwecken zugeführt zu werden. Dies wird unten anhand weiterer Ausführungsbeispiele näher erläutert.

Um dem Messsystem 110 ein neues Magazin 112 mit unverbrauchten Testelementen 122 zuzuführen, kann ein derartiges Magazin 112 beispielsweise einer Primärverpackung (beispielsweise einer Blisterverpackung) entnommen werden. Dabei können die Öffnungen 128, 130 zusätzlich durch Siegelfolien versiegelt sein. In diesem Fall kann auch auf die Umverpackung (beispielsweise die Blisterverpackung) verzichtet werden.

Vor dem Einschieben des Magazins 112 in den Magazinschacht 126 können dann diese Siegelfolien von Öffnungen 128, 130 entfernt werden. Dabei ist der Innenraum des Magazins 112 kurzfristig der Atmosphäre ausgesetzt. Um die dabei eindringende Feuchtigkeit aufzunehmen, kann im Inneren des Magazins 112 ein Trockenmittel gelagert sein, wie oben beschrieben, welches diese Feuchtigkeit problemlos aufnehmen kann.

Nach dem Einsetzen des Magazins 112 in den Magazinschacht 126 wird dann der Verschluss 116 betätigt, und das geräteseitige Dichtelement 118 wird in die Öffnungen 128, 130 eingeschoben. Der in Figur 2 dargestellte geschlossene Zustand ist der Zustand, in welchem sich das Messsystem 110 befindet, wenn dieses nicht verwendet wird. Um beim erstmaligen Einsetzen eines frischen Magazins 112 und der anschließenden Verriegelung durch das geräteseitige Dichtelement 118 einen ungenutzten Verlust des obersten Testelements 122 zu vermeiden, kann das oberste Testelement in jedem neuen Magazin 112 beispielsweise auch als "Dummy" ausgestaltet sein, beispielsweise in Form eines kostengünstigen Kunststoff- oder Pappteils, welches beim erstmaligen Einsetzen ausgestoßen und entsorgt wird.

Die Magazine 112 können beispielsweise als Einwegmagazine ausgestaltet sein, oder können einer Wiederverwertung zugeführt werden. Vorteilhaft an dieser Ausgestaltung der Dichtelemente 118, 132 ist auch, dass die durch diese Dichtelemente 118, 132 gebildete Dichtung mit jedem Magazin 112 ausgetauscht werden kann, wohingegen aufwändigere Teile, wie beispielsweise der Verschluss 116, im Analysegerät 114 verbleiben können und somit wiederverwertet werden können.

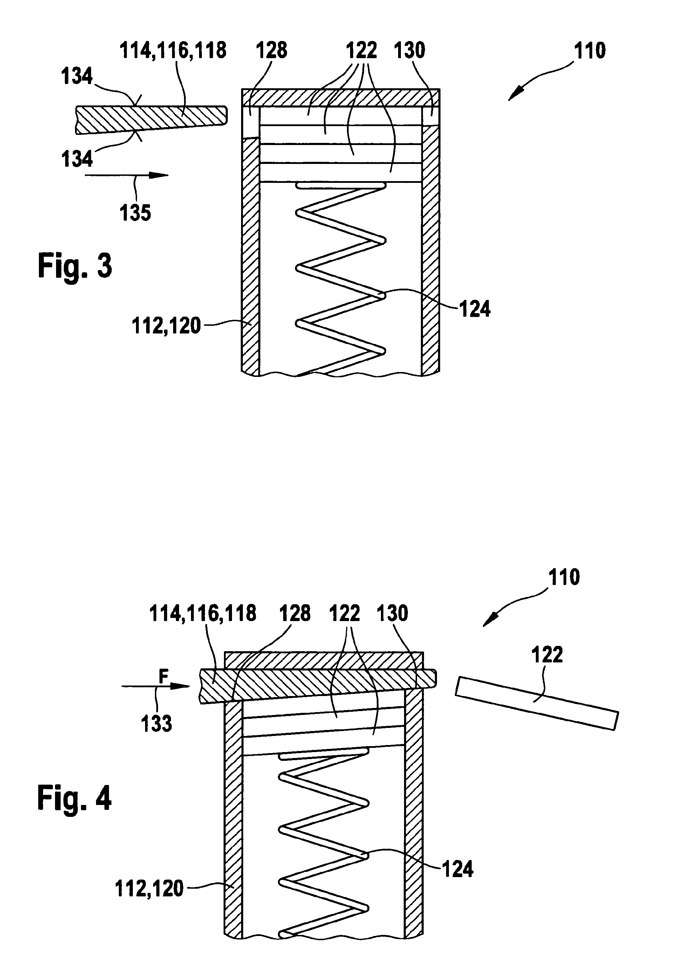

Bei dem in den Figuren 1 und 2 dargestellten Ausführungsbeispiel des Messsystems 110 hat das (geräteseitige) Dichtelement 118 einen sich in axiale Richtung im Wesentlichen nicht verändernden Querschnitt. Dementsprechend ist es bei diesem Ausführungsbeispiel bevorzugt, wenn dieses Dichtelement 118 mit den magazinseitigen Dichtelementen 132 zusammenwirkt. Die Herstellung dieser magazinseitigen Dichtelemente 132 kann jedoch die Kosten der Magazine 112 erhöhen. Dementsprechend ist in den Figuren 3 und 4 ein zweites Ausführungsbeispiel eines Messsystems 110 dargestellt, welches im Wesentlichen dem Ausführungsbeispiel gemäß den Figuren 1 und 2 entspricht.

Im Unterschied hierzu ist im Ausführungsbeispiel gemäß den Figuren 3 und 4 jedoch ein "konisches" Dichtelement 118 vorgesehen. Dieses konische Dichtelement 118 ist dabei derart ausgestaltet, dass es sich in seinem Querschnitt in Richtung der Einschubrichtung (in Figur 3 symbolisch durch den Pfeil 135 dargestellt) in seinem Querschnitt verjüngt. Beispielsweise kann das Dichtelement sich im eingeschobenen Zustand zwischen den Öffnungen 128 und 130 um 10 bis 90 % in seinem Querschnitt verjüngen, wobei typische Verjüngungen im Bereich zwischen 30 und 50 % liegen. Dabei sollte jedoch stets gewährleistet sein, dass auch im geöffneten Zustand gemäß Figur 3 das zweitoberste Verbrauchselement 122 nicht aus der Öffnung 128 herausgleiten kann und innerhalb des Magazins 112 gehalten wird. Dementsprechend sollte die Öffnung 128 (und damit auch der entsprechende Querschnitt des Dichtelements 118) kleiner sein als die doppelte Dicke eines Verbrauchselements 122.

Diese Verjüngung bzw. konische Ausgestaltung des Dichtelements 118 bewirkt, dass die Einschubkraft, welche in Figur 4 symbolisch mit der Bezugsziffer 133 bezeichnet ist, eine Komponente senkrecht zur Dichtfläche 134 aufweist, so dass das Dichtelement 118 gegen das Magazingehäuse 120 im Bereich der Öffnungen 128, 130 gepresst wird. Durch dieses Anpressen wird die Dichtwirkung erheblich verbessert. Ansonsten entspricht der Aufbau und die Funktionsweise des Messsystems 110 gemäß den Figuren 3 und 4 im Wesentlichen den Figuren 1 bzw. 2.

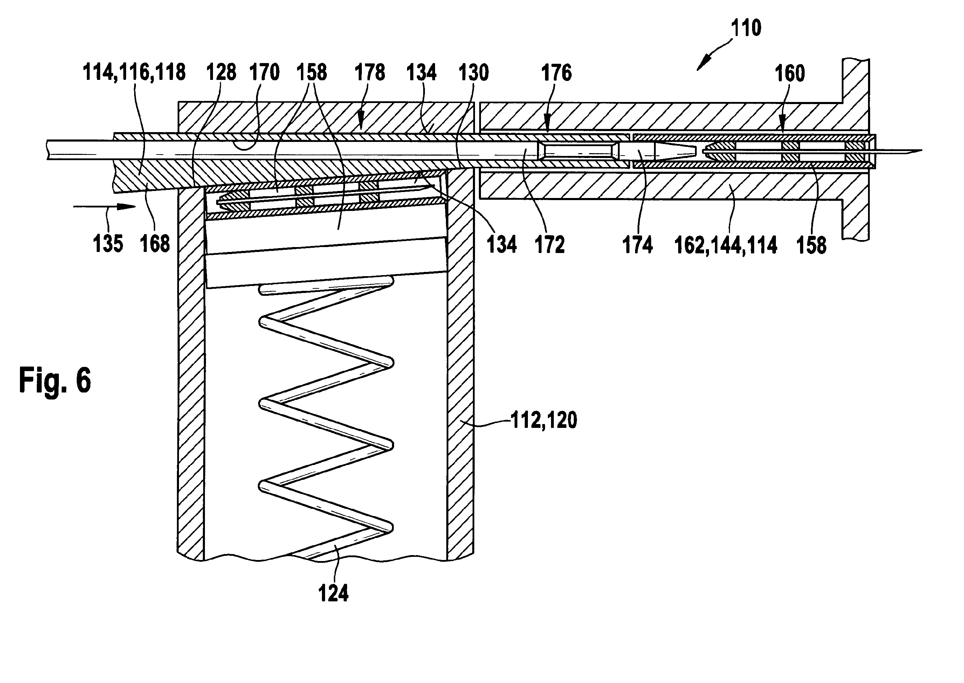

Dabei ist es jedoch nicht zwingend, dass die Konizität des Dichtelements 118 gleichmäßig über die gesamte Länge des Dichtelements 118 (bzw. Dichtschiebers) erfolgt. Es kann auch eine Ausgestaltung des Dichtelements 118 mit unterschiedlichen Abschnitten entlang seiner exaxialen Erstreckung vorliegen. Beispielsweise kann zunächst ein prismatischer Teil dazu dienen, das Verbrauchselement 122 aus dem Magazin 112 heraus und weiter in eine aktive Position zu schieben. Erst in einem letzten Teil der Länge des Dichtelements 118 wäre dieses dann konisch und dient dem Verschluss der Magazinöffnungen 128, 130 (siehe beispielsweise das Ausführungsbeispiel unten in Figur 6). In diesem Fall wäre ebenfalls das Magazin 112 für die kurze Messzeit geöffnet, wäre jedoch während der langen Zeit zwischen zwei Messungen geöffnet.

Eine ähnliche, in axiale Richtung ungleichförmige Querschnittsgestaltung des Dichtelements 118 ließe sich auch mit dem Ausführungsbeispiel gemäß den Figuren 1 und 2 kombinieren, also mit den zusätzlichen magazinseitigen Dichtelementen 132 (beispielsweise in Form von Elastomerdichtungen). In diesem Fall könnte der Dichtschieber bzw. das Dichtelement 118 in seinem vorderen Teil dünner in seinem Querschnitt ausgestaltet sein. Erst wenn das Dichtelement 118 ganz, also auch mit seinem mit größerem Querschnitt ausgestalteten hinteren Teil in das Magazin 112 eingeschoben ist, während die Öffnungen 128, 130 dicht verschlossen.

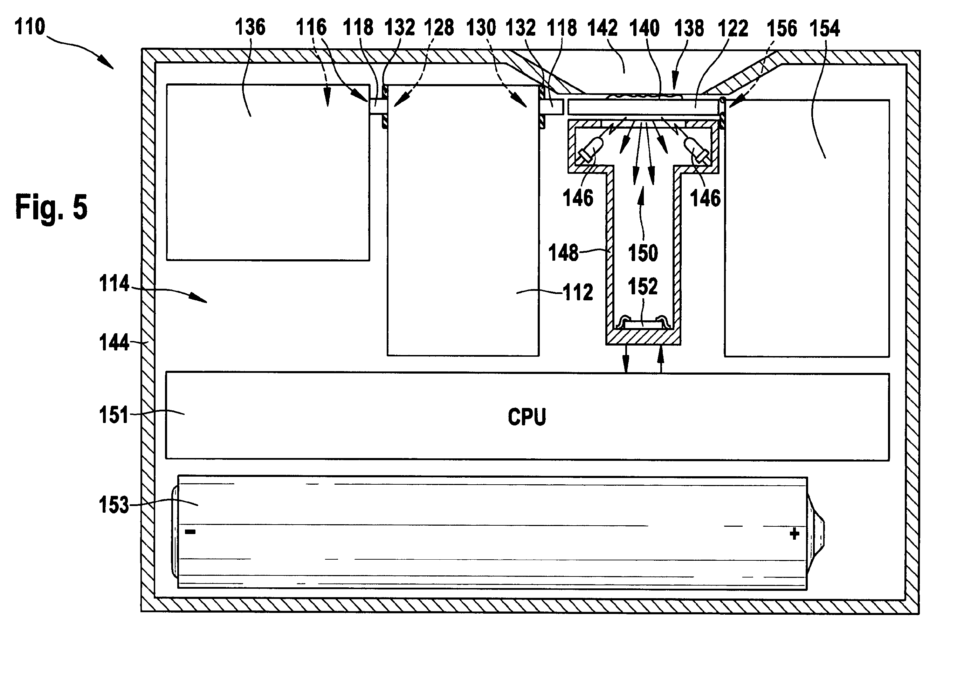

Während in den Figuren 1 bis 4 das eigentliche Analysegerät 114 jeweils lediglich angedeutet ist, ist in Figur 5 ein vollständiges Messsystem 110 dargestellt, welches ein vollständiges Analysegerät 114 mit einem darin aufgenommenen Magazin 112 darstellt. Das Magazin 112 ist beispielsweise ausgestaltet wie in dem Ausführungsbeispiel gemäß den Figuren 1 und 2. Dabei stellt Figur 5 lediglich eine schematisch vereinfachte Darstellung des Messsystems 110 dar, wobei zusätzliche Elemente vorhanden sein können, beispielsweise zusätzliche Halte- und Verriegelungselemente für das Magazin 112 (wie beispielsweise ein Magazinschacht 126).

Das Analysegerät 114 umfasst einen Verschluss 116, welcher neben dem Dichtelement 118 (welches in diesem Fall beispielsweise ausgestaltet ist wie in Figur 1 oder 2 dargestellt) einen Antrieb 136 für das Dichtelement 118. Mittels dieses Antriebs 136 lässt sich das Dichtelement 118 linear in der (in Figur 5 nicht dargestellten, vgl. beispielsweise Figur 3) Einschubrichtung 135 oder entgegengesetzt dieser Einschubrichtung 135 bewegen. Dadurch lässt sich das Dichtelement 118 durch die erste Öffnung 128, welche wiederum in diesem Ausführungsbeispiel mit den magazinseitigen Dichtelementen 132 ausgestattet ist, ins Innere des Magazins 112 einschieben. Dabei kann weitgehend auf die obige Beschreibung zu den Figuren 1 und 2 verwiesen werden. Der Antrieb 136 kann beispielsweise mechanische Antriebe und/oder elektromechanische Antriebe umfassen, wie sie dem Fachmann bekannt sind.

Durch das Einschieben in die erste Öffnung 128 wird, wie beispielsweise in Figur 2 dargestellt, ein Testelement 122 aus der (ebenfalls mit magazinseitigen Dichtelementen 132 ausgestatteten) zweiten Öffnung 130 ausgeschoben. Dieses Ausschieben erfolgt mittels des Dichtelements 118 so weit, dass das Testelement 122 in eine Messposition 138 überführt wird. In dieser Messposition 138 kann von außen eine flüssige Probe 140 (welche in Figur 5 lediglich angedeutet ist) durch eine Applikationsöffnung 142 im Gehäuse 144 des Analysegeräts 114 auf das Testelement 122 in der Messposition 138 aufgebracht werden. Die Applikationsöffnung 142 ist dabei beispielsweise derart ausgestaltet, dass ein Daumen mit einem darauf befindlichen Blutstropfen auf das Testelement 122 aufgedrückt werden kann, um den Blutstropfen auf das Testelement 122 zu übertragen.

Bei dem Ausführungsbeispiel in Figur 5 handelt es sich bei dem Testelement 122 beispielsweise um einen optischen Teststreifen. Bei diesem optischen Teststreifen löst die flüssige Probe 138 (wenn diese den Analyten enthält) eine Farbreaktion aus, beispielsweise indem der nachzuweisende Analyt mit einem entsprechenden Reagenz in dem Teststreifen 122 reagiert.

Diese Farbreaktion kann beispielsweise mittels entsprechender Leuchtdioden 146 in einem Detektor 148 des Analysegeräts 114 detektiert werden. Diese Leuchtdioden 146 (wobei auch andere Anregungsquellen verwendet werden können) bestrahlen das Testelement 122 mit Anregungslicht. Durch diese Anregung wird beispielsweise im Testelement 122 eine Fluoreszenz generiert, deren Vorhandensein und/oder Intensität von der Anwesenheit bzw. Konzentration des nachzuweisenden Analyten abhängt. Dieses Fluoreszenzlicht, welches in Figur 5 symbolisch mit der Bezugsziffer 150 bezeichnet ist, wird von einem optischen Detektor 152 (beispielsweise einer Photodiode) aufgenommen. Die dabei generierten Signale werden vom optischen Detektor 152 an eine Ansteuer- und Auswerteeinheit 151 (in Figur 5 symbolisch mit "CPU" bezeichnet, übermittelt. Aus diesen Signalen lässt sich dann in der Ansteuer- und Auswerteeinheit 151 auf die Anwesenheit bzw. Konzentration des nachzuweisenden Analyten in der flüssigen Probe 140 schließen.

Es sei darauf hingewiesen, dass die Detektionsmethode, wie sie anhand des Beispiels in Figur 5 beschrieben wurde, lediglich beispielhaft zu verstehen ist. Anstelle von Fluoreszenzlicht können beispielsweise auch reflektierte Lichtanteile, Phosphoreszenzanteile oder andere Arten von Licht detektiert werden. Auch können zusätzliche elektrische, mechanische, elektromechanische oder optische Komponenten in dem Analysegerät 114 vorgesehen sein, welche in Figur 5 nicht dargestellt sind. Das gesamte Analysegerät 114 wird in dem Ausführungsbeispiel gemäß Figur 5 durch eine Batterie 153 mit Energie versorgt. Auch andere Ausführungen sind jedoch denkbar, beispielsweise eine Stromversorgung über ein Kabel, eine drahtlose Stromversorgung, eine Stromversorgung durch andere Arten von Energiespeichern oder andere Arten von Energiezuführungen.

Das in Figur 5 dargestellte Analysegerät 114 lässt sich anstelle von optischen Testelementen 122 auch, alternativ oder zusätzlich, derart ausgestalten, dass mit anderen Arten von Testelementen 122 gearbeitet werden kann. Beispielsweise können zu diesem Zweck elektrochemische Testelemente 122 eingesetzt werden. Zu diesem Zweck kann beispielsweise in der Messposition 138 das Testelement 122 durch entsprechende elektrische Kontakte kontaktiert werden. Diese Kontakte können beispielsweise auch auf der Spitze des Dichtelements 118 vorgesehen sein.

Das Dichtelement 118 ist in Figur 5 derart ausgestaltet, dass dieses nicht nur das jeweils oberste Testelement 122 aus dem Inneren des Magazins 112 in die Messposition 138 überführen kann, sondern dass dieses auch darüber hinaus ein einmal gebrauchtes Testelement 122 aus der Messposition 138 in eine Entsorgungsposition überführen kann. Zu diesem Zweck kann das Analysegerät 114 beispielsweise eine Auswurföffnung als Entsorgungsöffnung aufweisen, durch welche das verbrauchte Testelement 122 aus dem Gehäuse 144 des Analysegeräts 114 ausgestoßen wird. Alternativ oder zusätzlich kann, wie in Figur 5 dargestellt, das Analysegerät 114 auch als Entsorgungsposition einen Abfallbehälter 154 aufweisen. Der Abfallbehälter 154 weist eine Entsorgungsöffnung 156 auf. Das Dichtelement 118 ist so in seiner Länge ausgestaltet, dass dieses das Testelement 122 aus der Messposition 138 durch die Entsorgungsöffnung 156 ins Innere des Abfallbehälters 154 einschieben kann. Der Abfallbehälter 154 kann dann in regelmäßigen Abständen oder bei Bedarf entleert werden, beispielsweise über eine (in Figur 5 nicht dargestellte) Klappe, und/oder indem der Abfallbehälter 154 dem Gehäuse 144 des Analysegeräts 114 entnommen wird. Auf diese Weise ist eine hygienische Entsorgung verbrauchter Testelemente 122 ohne ein Infektionsrisiko sichergestellt. Das Dichtelement 118 dient somit in dem in Figur 5 dargestellten Ausführungsbeispiel der dreifachen Funktion der Ausgabe eines Testelements 122 aus dem Magazin 112 in die Messposition 138, der Überführung des Testelements 122 aus der Messposition 138 in den Abfallbehälter 154 und der Abdichtung des Magazins 112.

In den bisher dargestellten Ausführungsbeispielen wurden als Verbrauchselemente Testelemente eingesetzt, welche dem Nachweis des mindestens einen Analyten in der mindestens einen Probe 140 dienen. Alternativ oder zusätzlich zu derartigen Testelementen 122 können die Verbrauchselemente jedoch auch andere Arten von Verbrauchselementen umfassen, beispielsweise Lanzettensysteme zum Perforieren einer Hautpartie. Beispiele derartige Messsysteme 110 sind in den Figuren 6 bis 9 dargestellt.

So zeigt Figur 6 ein Messsystem 110 in ähnlicher Darstellung und Ausgestaltung wie in Figur 4. In diesem Ausführungsbeispiel umfassen die Verbrauchselemente jeweils Lanzettensysteme 158, welche wiederum in einem Magazin 112 (welches wiederum als Reihen- oder Stangenmagazin ausgebildet ist), aufgenommen und durch eine Magazinfeder 124 mit Druck beaufschlagt sind. Wiederum weist das Magazin 112 zwei Öffnungen 128, 130 auf, wobei die erste Öffnung 128 wiederum, analog zu den Figuren 3 und 4, größer ausgestaltet ist als die zweite Öffnung 130. Ebenfalls analog zu den Figuren 3 und 4 kann ein konusförmiges Dichtelement in einer Einschubrichtung 135 ins Innere des Magazins 112 eingeschoben werden. Dabei wird das oberste der Lanzettensysteme 158 aus dem Inneren des Magazins 112 ausgeschoben und in eine Anwendungsposition 160 geschoben. Diese Anwendungsposition 160 ist aufgenommen in einer entsprechend ausgestalteten Führungsstruktur 162 im Gehäuse 144 des Analysegeräts 114.

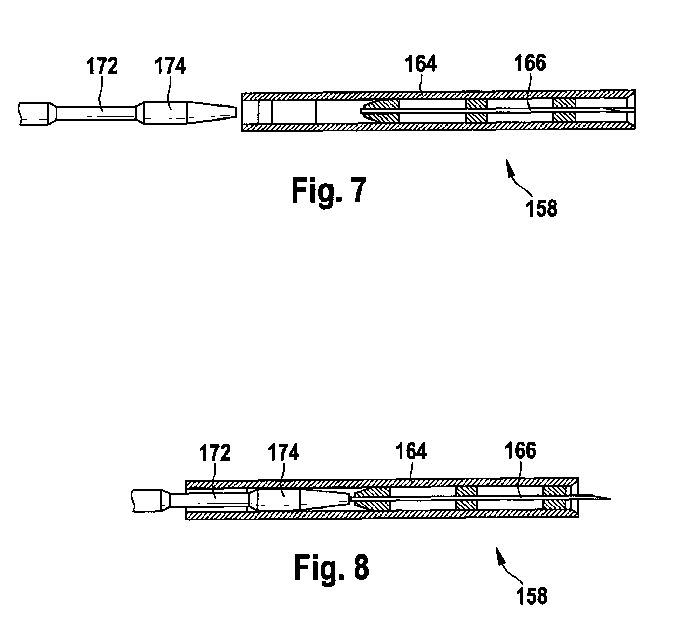

Das Lanzettensystem 158 ist in diesem Ausführungsbeispiel als Einweg-Lanzettensystem ausgestaltet und kann beispielsweise einem Design entsprechen, welches in EP 1 333 756 B1 beschrieben ist. Das Lanzettensystem 158 ist in den Figuren 7 und 8 im Detail dargestellt. Es umfasst ein Lanzettengehäuse 164, in welchem, gleitbar gelagert, eine Lanzettennadel 166 aufgenommen ist.

Wie in Figur 6 zu erkennen ist, weist das Dichtelement 118 in diesem Ausführungsbeispiel einen Mantel 168 auf, in welchem ein sich parallel zur Einschubrichtung 135 erstreckender Applikationskanal 170 aufgenommen ist. Dieser Applikationskanal 170 nimmt in diesem Ausführungsbeispiel einen gleitbar im Applikationskanal 170 gelagerten Antriebsstößel 172 auf. Dieser Antriebsstößel 172 ist an seinem vorderen Ende mit einem verdickten Bereich 174 ausgestattet, welcher in das Lanzettengehäuse 164 eindringt. Schnellt der Antriebsstößel 172 nach vorne, so wird die Lanzettennadel 166 innerhalb des Lanzettengehäuses 164 verschoben und dringt nach außen vor, um eine Perforation einer Hautpartie vorzunehmen. Für die weitere Funktionsweise der Lanzettensysteme 158 sowie für alternative Ausgestaltungen der Lanzettensysteme 158 kann auf die oben genannte EP 1 333 756 B1 verwiesen werden.

Des Weiteren ist bei dem in Figur 6 dargestellten Ausführungsbeispiel das Dichtelement 118 nicht in allen Bereichen gleichförmig ausgebildet. So ist das Dichtelement 118 in seinem vorderen Bereich 176 prismatisch und mit einem konstanten Querschnitt ausgestattet. Daran schließt sich ein konischer Bereich 178 an, welcher, wie oben beschrieben, die Abdichtung der Öffnungen 128, 130 begünstigt. Die in Figur 6 dargstellte Stellung ist die verschlossene Stellung, in welcher das Magazin 112 durch das Dichtelement 118 im Wesentlichen dicht abgedichtet ist. Der Antriebsstößel 172 muss dabei im Applikationskanal 170 nicht abgedichtet aufgenommen sein, da der Mantel 168 das Magazin 112 allein mit seiner außen liegenden Dichtfläche 134 verschließen kann.

Nicht dargestellt sind in Figur 6 wiederum übrige Bestandteile des Analysegeräts 114. Insbesondere ist wiederum der Verschluss 116 nicht vollständig dargestellt, welcher wiederum, wie beispielsweise in Figur 5 beschrieben, einen Antrieb 136 umfassen kann. Dieser Antrieb 136 würde in diesem Fall vorzugsweise nicht nur einen linearen Antrieb für das Dichtelement 118 umfassen, sondern auch eine Antriebseinheit für den Antriebsstößel 172, so dass dieser unabhängig von dem Dichtelement 118 in bzw. gegen die Einschubrichtung 135 bewegt werden kann.

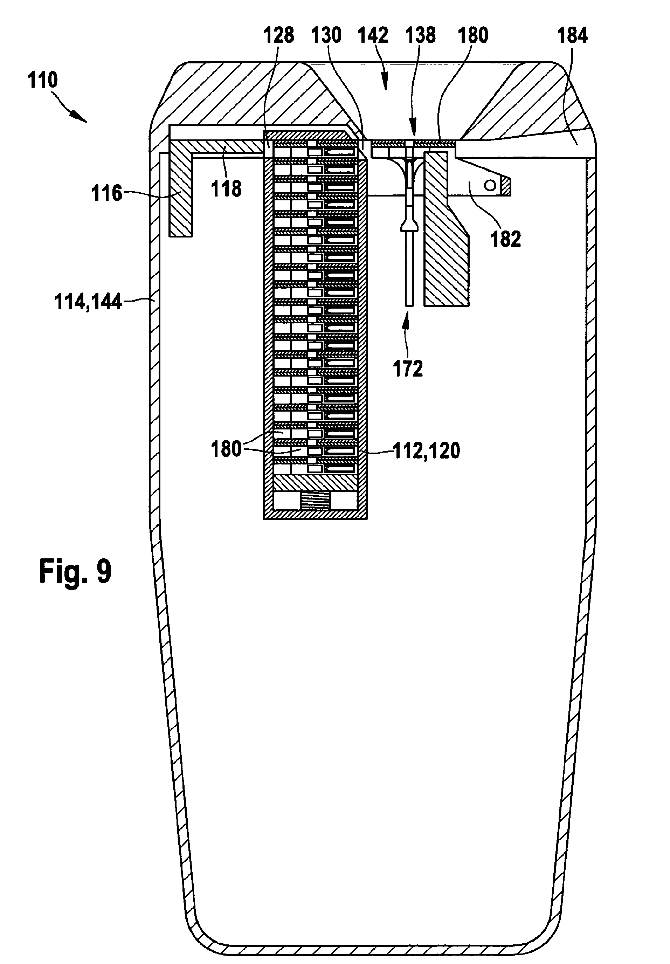

Bei dem Ausführungsbeispiel gemäß Figur 6 bewegt sich der Antriebsstößel 172 parallel zur Einschubeinrichtung 135 des Dichtelements 118. Dies ist jedoch nicht zwingend erforderlich, wie beispielsweise das Ausführungsbeispiel in Figur 9 zeigt. Dieses Ausführungsbeispiel eines Messsystems 110 stellt eine Abwandlung eines aus EP 1 203 563 A2 bekannten Messsystems dar, welches kombinierte Verbrauchselemente 180 verwendet.

Diese kombinierten Verbrauchselemente sind in einem Magazin 112 aufgenommen, welches beispielsweise wiederum als Stangen- oder Reihenmagazin ausgestaltet ist, analog zu den obigen Ausführungsbeispielen. Das Magazin 112 weist wiederum zwei Öffnungen 128, 130 auf. Durch die erste Öffnung 128 kann ein Dichtelement 118 in das Innere des Magazins 112 eingeschoben werden, um das oberste Verbrauchselement 180 aus dem Magazin in eine Messposition 138 zu schieben. Wiederum ist das Dichtelement 118 dabei derart ausgestaltet, dass dieses in der geschlossenen Position, das heißt wenn das Testelement ausgeschoben ist, in den Öffnungen 128, 130 verbleibt und diese dichtend verschließt. Nicht dargestellt ist in Figur 9 ein entsprechender Antrieb 136 des Verschlusses 116.

Die kombinierten Verbrauchselemente 180 sind dabei ausgestaltet wie in EP 1 203 563 A2 beschrieben und umfassen sowohl ein Testelement als auch ein Lanzettensystem. In der Messposition 138 klappt dabei ein Teil des Lanzettensystems nach unten und kann mittels eines Antriebsstößels 172 betätigt werden. Bei dieser Betätigung sticht die Lanzette senkrecht zur Flächenerstreckung des kombinierten Verbrauchselements 180 aus der Applikationsöffnung 142 heraus und perforiert dabei beispielsweise eine Hautpartie eines Fingers, welcher auf die Applikationsöffnung 142 gelegt ist. Der dabei gebildete Blutstropfen wird unmittelbar auf das in dem kombinierten Verbrauchselement 180 integrierte Testelement appliziert, um dort beispielsweise elektrochemisch oder optisch untersucht zu werden. Für weitere Details des kombinierten Verbrauchselements 180 kann auf die oben genannte Patentveröffentlichung verwiesen werden.

Wie oben beschrieben, verschließt das Dichtelement 118 im geschlossenen Zustand beide Öffnungen 128, 130. Das in EP 1 203 563 A2 beschriebene Messsystem weist dieses Merkmal nicht auf Darüber hinaus weist das in EP 1 203 563 A2 beschriebene System einen Auswerfer auf, welcher auch bei dem Ausführungsbeispiel in Figur 9 vorgesehen ist und dort mit der Bezugsziffer 182 bezeichnet ist. Dieser Auswerfer dient dazu, verbrauchte kombinierte Verbrauchselemente 180 durch eine Auswurföffnung 184 zu entsorgen. In einer Weiterentwicklung des Ausführungsbeispiels gemäß Figur 9 wird auf diesen Auswerfer 182 dadurch verzichtet, dass das Dichtelement 118 bzw. der Verschluss 116 derart ausgestaltet sind, dass diese, analog zum Beispiel gemäß Figur 5, das verbrauchte Verbrauchselement 180 auch gleich von der Messposition 138 durch die Auswurföffnung 184 ausstoßen. Auf diese Weise lassen sich die zusätzlichen mechanischen Elemente des Auswerfers 182 einsparen.

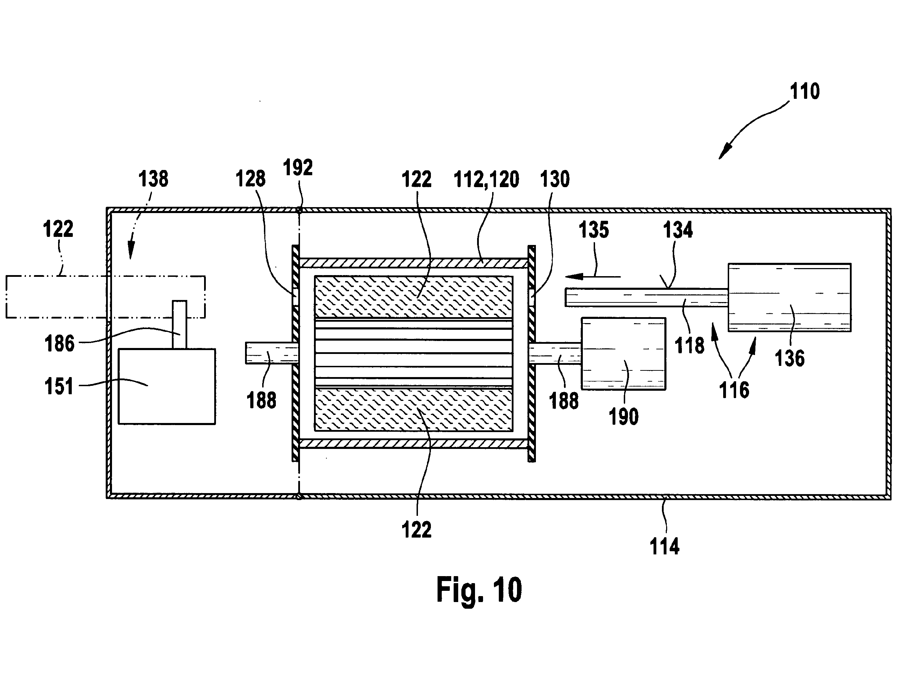

Bei den bislang beschriebenen Ausführungsbeispielen ist das Magazin 112 jeweils als Stangen- oder Reihenmagazin ausgestaltet. Figur 10 zeigt demgegenüber ein Ausführungsbeispiel eines Messsystems 110, bei welchem das Magazin 112 als Trommelmagazin ausgestaltet ist. Wiederum ist dieses Messsystem 110 lediglich schematisiert dargestellt, um die wesentlichsten Bauelemente und Funktionen aufzuzeigen.

Das Messsystem 110 weist wiederum ein Analysegerät 114 auf, wobei in diesem Fall wieder angenommen sei, dass es sich bei diesem Analysegerät 114 um ein Blutzuckermessgerät handelt. Die Testelemente 122 sind hier beispielhaft als elektrochemische Teststreifen ausgebildet. Zu diesem Zweck weist das Analysegerät 114 wiederum eine Messposition 138 auf, in welcher die Testelemente 122 durch eine elektrische Kontaktierung 186 kontaktiert werden können und mit einer flüssigen Probe (nicht dargestellt) beaufschlagt werden können. Das Analysegerät 114 weist weiterhin eine Ansteuer- und Auswertevorrichtung 150 auf, welche in Figur 10 ebenfalls stark schematisiert dargestellt ist. Daneben können noch weitere Vorrichtungen vorgesehen sein, beispielsweise ein Mikrocomputer zum Auswerten, Bedienelemente, Ausgabeelemente oder ähnliches, wie dies auch beispielsweise zusätzlich in dem Ausführungsbeispiel gemäß Figur 5 oder gemäß Figur 9 der Fall sein kann.

In diesem Beispiel gemäß Figur 10 ist wiederum ein Magazin 112 vorgesehen, welches jedoch, wie oben beschrieben, im Unterschied zu den obigen Ausführungsbeispielen als Trommelmagazin ausgestaltet ist. In diesem Trommelmagazin 112 können die Testelemente 122 beispielsweise sternförmig und symmetrisch zur Trommelachse gelagert sein. Beispielsweise können zu diesem Zweck einzelne Kammern innerhalb des Trommelmagazins 112 vorgesehen sein, in welchen die Testelemente 122 voneinander abgeschottet gelagert sind. Alternativ kann das Innere des Magazins 112 auch als eine einzige große Kammer ausgestaltet sein, wobei alle Testelemente 122 in derselben Kammeratmosphäre gelagert sind.

Dementsprechend kann auch eine Lagervorrichtung vorgesehen sein, welche in Figur 10 ebenfalls nicht dargestellt ist. Diese Lagervorrichtung kann beispielsweise mit entsprechenden Achsen 188 zusammenwirken, mittels derer das Magazin 112 beispielsweise über einen Motor 190 gedreht werden kann. In dem Magazin 112 kann weiterhin, was in Figur 10 ebenfalls nicht dargestellt ist, ein Trockenmittel aufgenommen sein, wie auch in den Magazinen 112 gemäß der vorhergehenden Ausführungsbeispiele.

Wiederum weist das trommelförmige Magazin 112 zwei Öffnungen 128, 130 in seinem Gehäuse 120 auf. Entsprechend ist, analog zu den obigen Ausführungsbeispielen, wiederum ein Verschluss 116 vorgesehen, welcher ein Dichtelement 118 mit entsprechenden Dichtflächen 134 aufweist. Über einen Antrieb 136 kann das Dichtelement 138 in die erste Öffnung 130 eingeschoben werden, so dass ein Testelement 122 aus der zweiten Öffnung 128 ausgestoßen wird und in die Messposition 138 überführt wird. Dort wird dieses Testelement 122 durch die elektrische Kontaktierung 186 kontaktiert und es kann eine Messung durchgeführt werden. In dieser Stellung ist das Dichtelement 118, im Gegensatz zu der in Figur 10 dargestellten geöffneten Stellung (welche lediglich kurzfristig eingenommen wird), vollständig in die Öffnungen 128, 130 eingefahren und verschließt diese. Die Öffnungen 128, 130 können zu diesem Zweck wiederum mit zusätzlichen magazinseitigen Dichtelementen 132 ausgestattet sein, welche in Figur 10 nicht dargestellt sind. Alternativ oder zusätzlich kann das Magazingehäuse 120 im Bereich dieser Öffnungen 128, 130 auch durch einen zumindest teilweisen verformbaren Kunststoff ausgestaltet sein, in welchen das Dichtelement 118 durch eine Presspassung eingepresst wird, um das Innere des Magazins 112 zu verschließen. Alternativ oder zusätzlich kann wiederum, wie oben beschrieben, das Dichtelement 118 auch zumindest teilweise konusförmig ausgestaltet sein, um die Dichtwirkung zu verbessern. Weiterhin kann, analog zu dem Ausführungsbeispiel gemäß Figur 5 oder Figur 6, das Dichtelement 118 auch derart ausgestaltet sein, dass dies nach Durchführung einer Messung in der Messposition 138 das verbrauchte Testelement 122 aus dem Analysegerät 114 ausstößt.

Vorteilhaft der in Figur 10 dargestellten Ausführungsform ist, dass das Magazin 112 vergleichsweise einfach ausgestaltet werden kann und dass alle Dichtungs- und/oder Aktivierungsteile für die Einzelelemente im Wesentlichen dem Analysegerät 114 zugeordnet werden können. Dies reduziert die Kosten für das Verbrauchsmaterial erheblich. Weiterhin fällt die Schnurlänge der Dichtung erheblich kleiner aus als in dem Fall, in welchem der gesamte Magazinschacht 126 abgedichtet würde (wobei die Dichtung der Ladeöffnung des Öffnungsmechanismus 192 am Analysegerät 114 selbst vorgesehen werden müsste), so dass bei gegebener Flächenpressung der Dichtung die erforderliche Gesamtkraft beim Schließen des Öffnungsmechanismus 192 nach Einlegen eines neuen Magazins 112 erheblich reduziert werden kann. Dadurch wird die Handhabung des Analysegeräts 114 vereinfacht, und die Struktur des Analysegeräts 114 und des Magazins 112 kann erheblich mechanisch entlastet werden.

### Bezugszeichenliste

- 110: Messsystem
- 112: Magazin
- 114: Analysegerät
- 116: Verschluss
- 118: (geräteseitiges) Dichtelement
- 120: Magazingehäuse
- 122: Testelemente
- 124: Magazinfeder
- 126: Magazinschacht
- 128: Öffnung
- 130: Öffnung
- 132: magazinseitige Dichtelemente
- 133: Einschubkraft
- 134: Dichtflächen
- 135: Einschubrichtung
- 136: Antrieb
- 138: Messposition
- 140: flüssige Probe
- 142: Applikationsöffnung
- 144: Gehäuse
- 146: Leuchtdioden
- 148: Detektor
- 150: Fluoreszenzlicht
- 151: Ansteuer- und Auswerteeinheit
- 152: optischer Detektor
- 153: Batterie
- 154: Abfallbehälter
- 156: Entsorgungsöffnung
- 158: Lanzettensysteme
- 160: Anwendungsposition
- 162: Führungsstruktur
- 164: Lanzettengehäuse
- 166: Lanzettennadel
- 168: Mantel
- 170: Applikationskanal
- 172: Antriebsstößel
- 174: verdickter Bereich
- 176: Bereich mit konstantem Querschnitt
- 178: konischer Bereich
- 180: kombiniertes Verbrauchselement
- 182: Auswerfer
- 184: Auswerföffnung
- 186: elektrische Kontaktierung
- 188: Achsen
- 190: Motor
- 192: Öffnungsmechanismus

## Patentansprüche

1. Messsystem (110), umfassend ein Analysegerät (114) mit mindestens einer Messfunktion und/oder einer Probennahmefunktion, sowie weiterhin umfassend mindestens ein Magazin (112) zur Aufnahme mindestens eines Verbrauchselements (122; 158; 180), wobei das Magazin (112) ein Magazingehäuse (120) mit mindestens einer Öffnung (128, 130) aufweist, wobei das Magazin (112) als auswechselbares, mit dem Analysegerät (114) verbindbares Magazin (112) ausgestaltet ist, wobei das Analysegerät (114) einen Verschluss (116) mit mindestens einem Dichtelement (118) aufweist, **dadurch gekennzeichnet, dass** das Dichtelement (118) eingerichtet ist, um mindestens ein Verbrauchselement (122; 158; 180) aus der Öffnung (128, 130) auszugeben, wobei das Dichtelement (118) einen Schieber oder Stößel umfasst, welcher in eine erste Öffnung (128) des Magazins (112) einschiebbar ist, um dann ein Verbrauchselement (122; 158; 180) aus einer zweiten Öffnung (130) auszustoßen, wobei das Dichtelement (118) in einem geschlossenen Zustand des Magazins (112) in der Öffnung (128, 130) verbleibt und diese verschließt.

2. Messsystem (110) gemäß dem vorhergehenden Anspruch, wobei das Dichtelement (118) und/oder die Öffnung (128, 130) zumindest teilweise verformbar, insbesondere elastisch, ausgestaltet sind.

3. Messsystem (110) gemäß dem vorhergehenden Anspruch, wobei das Dichtelement (118) eine Dichtfläche (134) aufweist und wobei die Öffnung (128, 130) ein verformbares magazinseitiges Dichtelement (132) aufweist.

4. Messsystem (110) gemäß einem der vorhergehenden Ansprüche, wobei das Dichtelement (118) in einer Einschubsrichtung (135) zumindest teilweise einen sich verjüngenden Querschnitt aufweist.

5. Messsystem (110) gemäß einem der vorhergehenden Ansprüche, wobei der Verschluss (116) eingerichtet ist, um das Verbrauchselement (122; 158; 180) mittels des Dichtelements (118) aus dem Magazin (112) in eine Mess- oder Anwendungsposition (138; 160) zu befördern.

6. Messsystem (110) gemäß dem vorhergehenden Anspruch, wobei der Verschluss (116) weiterhin eingerichtet ist, um das Verbrauchselement (122; 158; 180) mittels des Dichtelements (118) aus der Mess- oder Anwendungsposition (138; 160) in eine Entsorgungsposition (184) und/oder eine Entsorgungsvorrichtung (154) zu befördern.

7. Messsystem (110) gemäß einem der vorhergehenden Ansprüche, wobei das Dichtelement (118) einen Mantel (168) mit mindestens einer Dichtfläche (134) und mindestens einen axial im Dichtelement (118) verlaufenden Applikationskanal (170) umfasst.

8. Messsystem (110) nach dem vorhergehenden Anspruch, wobei in dem Applikationskanal (170) mindestens ein elektrischer Kontakt zur Kontaktierung eines Verbrauchselements (122; 158; 180) aufgenommen ist.

9. Messsystem (110) nach einem der beiden vorhergehenden Ansprüche, wobei in dem Applikationskanal eine optische Vorrichtung aufgenommen ist.

10. Messsystem (110) nach einem der drei vorhergehenden Ansprüche, wobei in dem Applikationskanal eine mechanische Vorrichtung, insbesondere ein Antriebsstößel (172) für ein Lanzettensystem (158), aufgenommen ist.

11. Messsystem (110) gemäß einem der vorhergehenden Ansprüche, wobei das Verbrauchselement (122; 158; 180) mindestens eines der folgenden Elemente umfasst: ein Testelement (122), insbesondere einen Teststreifen, insbesondere einen elektrochemischen und/oder optischen Teststreifen; ein Probennahmeelement, insbesondere ein Lanzettensystem (158), insbesondere ein Einweglanzettensystem; ein kombiniertes Verbrauchselement (180), insbesondere ein ein Lanzettensystem und ein Testelement umfassendes kombiniertes Verbrauchselement.

12. Messsystem (110) gemäß einem der vorhergehenden Ansprüche, wobei mindestens ein erstes Verbrauchselement (122; 158; 180) als Dummy ausgestaltet ist.

13. Messsystem (110) gemäß einem der vorhergehenden Ansprüche, wobei das Analysegerät (114) eine Magazinaufnahme aufweist, insbesondere einen Magazinschacht (126) oder ein Magazinfach, welches eingerichtet ist, um das Magazin (112) aufzunehmen.

14. Messsystem (110) gemäß einem der vorhergehenden Ansprüche, wobei das Magazin (112) ein Reihen- oder Stangenmagazin mit einer Magazinfedcr (124) umfasst.

15. Messsystem (110) gemäß einem der vorhergehenden Ansprüche wobei das Magazin (112) ein Trommelmagazin umfasst.

## Claims

1. Measuring system (110), comprising a testing device (114) with at least one measuring function and/or a sampling function, further comprising at least one magazine (112) for accommodating at least one consumable element (122; 158; 180), which magazine (112) has a magazine housing (120) with at least one opening (128, 130), and the magazine (112) is designed as a replaceable magazine (112) which can be connected to the testing device (114), which testing device (114) has a closure (116) with at least one seal element (118), **characterised in that** the seal element (118) is designed so that at least one consumable element (122; 158; 180) can be dispensed from the opening (128, 130), and the seal element (118) has a plunger or ram which can be pushed into a first opening (128) of the magazine (112) so as to then eject a consumable element (122; 158; 180) from a second opening (130), and when the magazine (112) is in a closed state, the seal element (118) remains in the opening (128, 130) and closes it off.

2. Measuring system (110) as claimed in the preceding claim, in which the seal element (118) and/or the opening (128, 130) are of an at least partially deformable, in particular elastic, design.

3. Measuring system (110) as claimed in the preceding claim, in which the seal element (118) has a seal surface (134) and the opening (128, 130) has a deformable magazine-side seal element (132).

4. Measuring system (110) as claimed in one of the preceding claims, in which the seal element (118) has an at least partially converging cross-section in a pushing-in direction (135).

5. Measuring system (110) as claimed in one of the preceding claims, in which the closure (116) is designed so that the consumable element (122; 158; 180) is moved out of the magazine (112) into a measuring or application position (138; 160) by means of the seal element (118).

6. Measuring system (110) as claimed in the preceding claim, in which the closure (116) is additionally designed so that the consumable element (122; 158; 180) is moved out of the measuring or application position (138; 160) into a disposal position (184) and/or a disposal device (154) by means of the seal element (118).

7. Measuring system (110) as claimed in one of the preceding claims, **characterised in that** the seal element (118) comprises a casing (168) with at least one seal surface (134) and at least one application passage (170) extending axially through the seal element (118).

8. Measuring system (110) as claimed in the preceding claim, in which at least one electrical contact is accommodated in the application passage (170) for contacting a consumable element (122; 158; 180).

9. Measuring system (110) as claimed in one of the two preceding claims, in which an optical device is accommodated in the application passage.

10. Measuring system (110) as claimed in one of the three preceding claims, in which a mechanical device, in particular a driving ram (172) for a lancet system (158), is accommodated in the application passage.

11. Measuring system (110) as claimed in one of the preceding claims, in which the consumable element (122; 158; 180) is at least one of the following elements: a test element (122), in particular a test strip, in particular an electrochemical and/or optical test strip; a sampling element, in particular a lancet system (158), in particular a disposable lancet system; a combined consumable element (180), in particular a combined consumable element comprising a lancet system and a test element.

12. Measuring system (110) as claimed in one of the preceding claims, in which at least a first consumable element (122; 158; 180) is provided in the form of a dummy.

13. Measuring system (110) as claimed in one of the preceding claims, in which the testing device (114) has a magazine holder, in particular a magazine shaft (126) or a magazine compartment, which is designed to accommodate the magazine (112).

14. Measuring system (110) as claimed in one of the preceding claims, in which the magazine (112) comprises a serial or rod magazine with a magazine spring (124).

15. Measuring system (110) as claimed in one of the preceding claims, in which the magazine (112) is a drum magazine.

## Revendications

1. Système de mesure (110) comprenant un appareil d'analyse (114) comprenant au moins une fonction de mesure et/ou une fonction de prélèvement d'échantillon, et comprenant en outre au moins un magasin (112) pour la réception d'au moins un élément de consommation (122 ; 158 ; 180), le magasin (112) présentant un logement de magasin (120) comportant au moins une ouverture (128 ; 130), le magasin (112) étant réalisé sous la forme de magasin échangeable, apte à être relié à l'appareil d'analyse (114), l'appareil d'analyse (114) présentant une fermeture (116) comprenant au moins un élément d'étanchéité (118), **caractérisé en ce que** l'élément d'étanchéité (118) est conçu pour distribuer au moins un élément de consommation (122 ; 158 ; 180) hors de l'ouverture (128 ; 130), l'élément d'étanchéité (118) comprenant un coulisseau ou un poussoir qui peut venir s'insérer dans une première ouverture (128) du magasin (112) pour pouvoir expulser un élément de consommation (122 ; 158 ; 180) hors d'une deuxième ouverture (130), l'élément d'étanchéité (118) subsistant, à l'état fermé du magasin (112), dans l'ouverture (128 ; 130) et fermant cette dernière.

2. Système de mesure (110) selon la revendication précédente, dans lequel l'élément d'étanchéité (118) et/ou l'ouverture (128 ; 130) étant réalisé pour pouvoir être au moins en partie déformé, en particulier de manière élastique.

3. Système de mesure (110) selon la revendication précédente, dans lequel l'élément d'étanchéité (118) présente une surface d'étanchéité (134) et l'ouverture (128 ; 130) présentant un élément d'étanchéité déformable, côté magasin.

4. Système de mesure (110) selon l'une quelconque des revendications précédentes, dans lequel l'élément d'étanchéité (118) présentant, dans une direction d'insertion par poussée (135), au moins en partie, une section transversale se rétrécissant.

5. Système de mesure (110) selon l'une quelconque des revendications précédentes, dans lequel la fermeture (116) est réalisée pour amener l'élément de consommation (122; 158; 180), au moyen de l'élément d'étanchéité (118), hors du magasin (112) dans une position de mesure ou d'utilisation (138 ; 160).

6. Système de mesure (110) selon la revendication précédente, dans lequel la fermeture (116) est réalisée en outre pour amener l'élément de consommation (122; 158; 180), au moyen de l'élément d'étanchéité (118) depuis la position de mesure ou d'utilisation (138; 160) dans une position d'évacuation (184) et/ou dans un dispositif d'évacuation (154).

7. Système de mesure (110) selon l'une quelconque des revendications précédentes, dans lequel l'élément d'étanchéité (118) comprend une enveloppe (168) comprenant au moins une surface d'étanchéité (134), et au moins un canal d'application (170) s'étendant en direction axiale dans l'élément d'étanchéité (118).

8. Système de mesure (110) selon la revendication précédente, dans lequel, dans le canal d'application (170), vient se disposer un contact électrique pour la mise en contact d'un élément de consommation (122 ; 158; 180).

9. Système de mesure (110) selon l'une quelconque des deux revendications précédentes, dans lequel, dans le canal d'application, vient se loger un dispositif optique.

10. Système de mesure (110) selon l'une quelconque des trois revendications précédentes, dans lequel, dans le canal d'application, vient se loger un dispositif mécanique, en particulier un poussoir d'entraînement (172) pour un système de lancettes (158).

11. Système de mesure (110) selon l'une quelconque des revendications précédentes, dans lequel l'élément de consommation (122 ; 158 ; 180) comprend au moins un des éléments suivants : un élément d'essai (122), en particulier une bandelette d'essai, en particulier une bandelette d'essai électrochimique et/ou optique ; un élément de prélèvement d'échantillon, en particulier un système de lancettes (158), en particulier un système de lancettes à jeter ; un élément de consommation combiné (180), en particulier un élément de consommation combiné comprenant un système de lancettes et un élément d'essai.

12. Système de mesure (110) selon l'une quelconque des revendications précédentes, dans lequel au moins un premier élément de consommation (122 ; 158 ; 180) est réalisé sous la forme d'un dummy.

13. Système de mesure (110) selon l'une quelconque des revendications précédentes, dans lequel l'appareil d'analyse (114) présente un logement de magasin, en particulier une gaine de magasin (126) ou un compartiment de magasin, qui est réalisé pour que vienne s'y loger le magasin (112).

14. Système de mesure (110) selon l'une quelconque des revendications précédentes, dans lequel le magasin (112) comprend un magasin à rangs ou à cartouches comportant un ressort de magasin (124).

15. Système de mesure (110) selon l'une quelconque des revendications précédentes, dans lequel le magasin (112) est un magasin à tambour.
